# EUROPÄISCHE PATENTSCHRIFT

(11) **EP 3 117 812 B1**
(45) Veröffentlichungstag und Bekanntmachung des Hinweises auf die Patenterteilung: **31.01.2018**
(21) Anmeldenummer: 15176716.7
(22) Anmeldetag: 14.07.2015
(51) Int. Cl.: A61F 13/496, A61F 13/15

(54) **WIEDERVERWENDBARE SCHWIMMWINDEL**
REUSABLE SWIMMING NAPPY
COUCHE ETANCHE REUTILISABLE

(43) Veröffentlichungstag der Anmeldung: 18.01.2017
(73) Patentinhaber: Lässig GmbH, 64832 Babenhausen (DE)
(72) Erfinder: LÄSSIG, Claudia, 63739 Aschaffenburg (DE)
(74) Vertreter: Patentanwälte Gierlich & Pischitzis Partnerschaft mbB

(56) Entgegenhaltungen:
- EP-A1- 2 918 253
- GB-A- 2 507 084
- US-A1- 2013 261 580
- US-B1- 6 797 856
- US-B1- 7 678 094

## Beschreibung

### Gebiet der Erfindung

Die Erfindung betrifft eine wiederverwendbare Schwimmwindel in Form eines Höschens, mit einem elastischen Abschluss im Taillen- und/oder Beinbereich.

### Stand der Technik

In den letzten Jahrzehnten wurden wiederverwendbare Stoffwindeln, die bspw. aus Baumwolle bestehen, bei der Verwendung durch stark absorbierende Wegwerfwindeln ersetzt.

Dies ist teilweise dem Umstand geschuldet, dass Wegwerfwindeln in der Regel viel mehr Feuchtigkeit aufnehmen können als bspw. Stoffwindeln, leichter zu verwenden sind und bei Verschmutzung leicht entsorgt werden können.

Allerdings sind weder wiederverwendbare Stoffwindeln noch herkömmliche Wegwerfwindeln für Säuglinge oder Kleinkinder geeignet, um mit diesen zu schwimmen oder an Wasseraktivitäten teilzunehmen.

So sind sowohl stark absorbierende Wegwerfwindeln als auch wiederverwendbare Stoffwindeln bspw. zu sperrig und sie nehmen zu viel Wasser auf, um mit ihnen zu schwimmen oder an Wasseraktivitäten teilzunehmen. Das z.T. erheblich höhere Gewicht nach Aufnahme großer Mengen an Wasser stellt zusätzlich ein Sicherheitsrisiko dar, da dadurch ein Säugling oder Kleinkind während des Schwimmens behindert wird und schlimmstenfalls sogar ertrinken kann.

Zusätzlich zu potentiellen Sicherheitsrisiken sind sowohl absorbierende Wegwerfwindeln als auch Stoffwindeln häufig unbequem, wenn sie sich mit Wasser vollsaugen.

Viele Eltern wechseln daher häufig die Windeln von Kindern, wenn diese bspw. zum Schwimmen oder Baden gehen. Etabliert haben sich dabei Schwimmwindeln die nicht aus stark wasserabsorbierenden Materialien wie bspw. Baumwolle bestehen.

Diese Schwimmwindeln für Kinder müssen mehrere Materialeigenschaften aufweisen. Zum einen müssen Materialien verwendet werden, die wasserdicht in dem Sinne sind, dass nicht Wasser von außen (z.B. Bade-/Meerwasser) in die Windel eindringt oder sich die Schwimmwindel damit vollsaugt. Gleichzeitig müssen Materialien verwendet werden, die Wasser bzw. flüssige Körperflüssigkeiten wie z.B. Urin aus der Schwimmwindel nach außen transportieren. Die eingesetzten Materialien sollten demnach zum einen atmungsaktiv sein, zum anderen eine Wasserdampfdurchlässigkeit aufweisen. Höherpreisige Produkte sollten darüber hinaus noch ein Feuchtigkeitsmanagement (engl. Wicking) aufweisen, d.h. Materialien sollten zum Einsatz kommen, die nicht nur wasserdicht sind und dennoch Wasserdampf durchlassen (sogenannte Atmungsaktivität/Wasserdampfdurchlässigkeit), vielmehr sollten Materialien verwendet werden, die zwar selbst nicht wasserdicht sind, aber die auftretende Feuchtigkeit (z.B. Schweiß oder Urin) schnell und aktiv vom Körper wegtransportieren (Feuchtigkeitsmanagement/Wicking). Mit Wicking wird demnach ein Effekt bezeichnet, bei dem Nässe auf eine größtmögliche Materialfläche ausgebreitet wird, um so die Verdunstung zu beschleunigen.

Wasserdampf kann bspw. auf zwei Arten durch wasserdichte Membranen gelangen. Zum einen durch sogenannte mikroporöse Membranen. Mikroporös heißt, dass die Membran mikroskopisch kleine Löcher hat, die so groß sind, dass Wasserdampfmoleküle hinaus können, aber gleichzeitig so klein sind, dass die größeren Wassermoleküle nicht hinein können. Als mikroporöse Membranen sind bspw. GoreTex® und eVENT® bekannt. Beide basieren auf einer gereckten Polytetrafluorethylen (ePTFE) Membran. Gereckt heißt, dass die Poren auf die Größe gebracht werden, dass Wasserdampf hindurch kann.

Das Problem bei solchen mikroporösen Membranen ist, dass Körperöle, Schweiß, aber auch Cremes von der Membran angesaugt werden und Kanäle im ePTFE hinterlassen, durch die Feuchtigkeit in die Produkte gelangen. Um das zu verhindern, können die Membrane mit bspw. einer dünnen Polyurethan (PU) Schicht überzogen werden. Dadurch werden die Membranen dauerhaft dicht. Gleichzeitig schränkt eine solche PU-Schicht die Atmungsaktivität jedoch ein, weil sie Feuchtigkeit absorbiert und hält - weswegen entsprechende Produkte sich bei körperlicher Anstrengung innen nass anfühlen. Der Schweiß kondensiert an der PU-Schicht, wird dort absorbiert und wird langsam nach außen gedrückt.

Alternativ können sogenannte geschlossenzellige Membrane zum Einsatz kommen. Bei diesen lagert sich die Feuchtigkeit auf der Innenseite an. Die Membran quillt auf und die Wasserdampfmoleküle werden durch die Membran transportiert - eine Art Osmose-Prinzip. Dies funktioniert mit einer kleinen Zeitverzögerung. Geschlossenzellige Membranen gelten als deutlich robuster, weil sie keine Poren haben, die sich vergrößern (undicht) oder verstopfen (keine Atmung mehr) können. Die meisten geschlossenzellige Membran-Systeme sind aus Polyester.

Darüber hinaus gibt es noch wasserdichte Beschichtungen. Derer gibt es sehr viele und in erster Linie handelt es sich um (z.T. mikroporöse) Polyurethanbeschichtungen. In allen Fällen muss ein Partialdruckgefälle bestehen, um den Wasserdampfdurchgang zu ermöglichen.

Alle nicht-wasserdichten Stoffe lassen auch Wasserdampfmoleküle hindurch. Hierbei besteht jedoch das Problem, dass viele der Stoffe den Wasserdampf aufnehmen und speichern. Je nachdem, wie lange der Stoff dann nass ist beziehungsweise wie schnell er wieder trocken ist, entscheidet dies über die Funktionalität der Ware. Baumwolle zieht bspw. Feuchtigkeit schnell weg vom Körper, jedoch gibt sie diese nicht wieder ab. Baumwolle speichert bis zu 60% des Eigengewichts an Nässe. Das ist doppelt ungünstig: Zum einen kühlt die Nässe den Körper schnell aus und zum anderen kann die Membran, selbst wenn diese atmungsaktiv ist, nicht funktionieren, wenn die Schicht(en) darunter die Nässe speichern, sodass diese nicht heraus kann. Alternative Materialien sind die Poly-Stoffe, die bei Normalklima (20° C) und 65% Luftfeuchtigkeit eine Feuchtigkeitsaufnahme von etwa 7% des Eigengewichts (Acryl; (AC)), 0,5% für Polyester (PES), 4,5% für Polyamid (PA) und 0% für Polypropylene (PP) aufweisen. Weil auch zwischen 0% und 7% Feuchtigkeitsaufnahme ein spürbarer Funktionsunterschied besteht, gelten PES und PP als atmungsaktive Materialien.

Bestimmte Strickkonstruktionen mit unterschiedlichen Ebenen können auf mechanischem Wege Feuchtigkeit nach außen leiten. Doppellagige Materialien, also Stoffe, die aus zwei Komponenten mit unterschiedlichen Eigenschaften bestehen, leiten die Nässe aktiver nach außen weg. Teilweise spürt man dann noch die Nässe auf der Außenlage, während innen ein trockenes Gefühl besteht. Wichtig bei guten Wicking-Stoffen ist die schnelle Verteilung der Nässe auf möglichst großer Fläche. Dadurch kann die Feuchtigkeit besser verdampfen bzw. großflächiger an die nächste Schicht weitergegeben werden.

All diese Materialeigenschaften müssen idealerweise miteinander kombiniert werden um den Bedürfnissen der Eltern und der Kinder bei wiederverwendbaren Schwimmwindeln zu entsprechen.

Aus der US7678094B1 ist eine mehrlagige wiederverwendbare Schwimmwindel bekannt, deren einzelne Lagen bestimmte Materialeigenschaften wie Atmungsaktivität, Wasserdampfdurchlässigkeit etc. aufweisen. Nachteilig an der beschriebenen Schwimmwindel ist, dass bspw. die einzelnen Lagen miteinander durch Nahtführung zusammengehalten werden, was zur Reibung auf der sehr sensitiven Kinderhaut führen kann, wodurch Rötungen und Schwellungen hervorgerufen werden können. Weiterhin werden in der hautzugewandten Lage zwar Materialien mit einem "Wicking"-Effekt verwendet, nicht jedoch wie bei der vorliegenden Erfindung die "one-directional-wicking" Materialkombinationen, die den Feuchtigkeitskontakt mit der Haut auf ein Minimum reduzieren und somit ein angenehmes und hautfreundliches Empfinden hervorruft.

### Aufgabe

Hiervon ausgehend liegt die Aufgabe der vorliegenden Erfindung darin, eine wiederverwendbare Schwimmwindel bereitzustellen, die einen hohen Tragekomfort aufweist und gleichzeitig Haut und Umwelt weitestgehend feuchtigkeitsfrei hält und somit das Einsatzgebiet derartiger Schwimmwindeln erweitert.

### Lösung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht. Die Erfindung umfasst auch alle sinnvollen und insbesondere alle erwähnten Kombinationen von unabhängigen und/oder abhängigen Ansprüchen.

Eine erfindungsgemäße wiederverwendbare Schwimmwindel ist in Form eines Höschens, mit einem elastischen Abschluss im Taillen- und/oder Beinbereich, gestaltet. Weiterhin weist sie auf:
a) eine Außenschicht, wobei die Außenschicht aus einer elastischen Maschenware oder einer Webware ausgestaltet ist;
b) eine wasserabsorbierende Zwischenschicht, wobei die wasserabsorbierende Zwischenschicht aus einem Textilgewebe ausgestaltet ist;
c) eine wasserdampfdurchlässige Innenschicht, wobei die wasserdampfdurchlässige Innenschicht aus einer Maschenware aus synthetischen Fasermaterialien ausgestaltet ist;
d) und wobei die wasserabsorbierende Zwischenschicht im Wesentlichen ohne Nahtführung zwischen Außen- und Innenschicht aufgebracht ist.

Der Ausdruck "elastischer Abschluss im Taillen- und/oder Beinbereich" umfasst sämtliche dem Fachmann bekannte Abschlüsse wie bspw. elastische Bünde, Stränge, Bänder, Filamente, Filamentwülste, Züge und dgl., die nahe bei einer Öffnung der wiederverwendbaren Schwimmwindel angeordnet sind, die ein Bein und/oder eine Taille eines Trägers aufnimmt. Ein bevorzugter elastischer Abschluss stellt bspw. einen Gummizug dar.

Dieser elastische Abschluss kann im Taillen- und/oder Beinbereich durch eine oder mehrere Nähte, bspw. zwei Nähte, an die wiederverwendbare Schwimmwindel befestigt werden.

Alternative Abschlüsse sind ebenfalls von der Erfindung umfasst. So können bspw. auch Knöpfe, Reiß- oder Klettverschlüsse und dgl. zum Einsatz kommen.

Die erfindungsgemäße Schwimmwindel weist eine Außenschicht auf, wobei die Außenschicht aus einer elastischen Maschenware oder einer Webware ausgestaltet ist.

Der Ausdruck "elastische Maschenware" umfasst sämtliche dem Fachmann bekannte textile Flächengebilde, bei denen eine mittels Faden gebildete Schleife in eine andere Schleife hineingeschlungen ist. Die auf diese Weise entstehenden Maschen können unter Verwendung eines oder mehrerer Fäden gebildet werden. Wirk- und Strickware gehören bspw. zu den Maschenwaren und sind erfindungsgemäß umfasst.

Der Vorteil von gewirkten Stoffen liegt in ihrer großen Elastizität. Für die erfindungsgemäße Schwimmwindel sind Gewirke daher hervorragend geeignet. Wolle und grobe Garne eignen sich zum Wirken ebenso wie sehr feine Garne. Beispiele für erfindungsgemäße elastische Maschenware sind bspw. elastische Maschenware aus Polyamid, Polypropylen, Polyester oder entsprechende Mischungen dieser Materialien, Elasthan (Block-Copolymer aus den Bestandteilen Polyurethan und Polyethylenglykol) oder anderen elastischen Fasern und deren Mischungen. Nylon-Gewirke und Gewirke, die unter Verwendung von elastischen Fasern hergestellt werden wie bspw. Pique, Fleece und Pannesamt.

Der Ausdruck "Webware" umfasst sämtliche dem Fachmann bekannte manuell oder maschinell gefertigten Erzeugnisse der Weberei wie bspw. Tuch, Samt, Velours, Plüsch, Frottee und sonstigen textilen Flächengebilden aus mindestens zwei rechtwinklig oder nahezu rechtwinklig verkreuzten Fadensystemen.

Gewebe sind bei hohen Fadendichten besonders widerstands- und strapazierfähig. Gesteigert wird dieser Effekt noch, wenn Zwirne anstelle von einfachen Garnen eingesetzt werden.

Gewebearten bzw. Webware, die erfindungsgemäß umfasst sind, können bspw. sein: Batist, Brokat, Chiffon, Chintz, Crepe (Krepp), Enoa, Damast, Denim, Drillich (Drell), Etamin (Siebtuch), Fil-a-fil, Flanell, Gabardine, Georgette, Jersey, Loden, Natte, Nessel, Pinpoint, Pique (Pikee), Popeline, Satin, Seersucker, Taft, Tuch, Tweed, Vollzwirn, Walkstoff.

Weiterhin besteht die Außenschicht in einer bevorzugten Ausführungsform aus wasserdichten Materialien oder Mischungen aus wasserdichten und wasserdurchlässigen Materialien.

In einer weiteren bevorzugten Ausführungsform zeichnet sich die wiederverwendbare Schwimmwindel dadurch aus, dass die Außenschicht eine Beschichtung und/oder eine Membran, vorzugsweise eine mikroporöse, hydrophobe und/oder hydrophile Membran, aufweist. Diese Beschichtung und/oder Membran kann mit weiteren Schichten flächig verbunden sein. Diese weiteren Schichten können ebenfalls Textilien, Folien aus Kunststoff oder Metall, Schaumstoff oder andere sein. Die Verbindung erfolgt stoffschlüssig beispielsweise durch Verkleben oder Verschmelzen auf speziellen Textilmaschinen (Lamination). Die Beschichtung und/oder die Membran soll insbesondere die Wasserdichtigkeit der wiederverwertbaren Schwimmwindel gewährleisten bzw. erhöhen.

Durch die Lamination entsteht ein Material, das die Eigenschaften seiner Ausgangsmaterialien kombiniert: So liefert bspw. ein kräftiges Gewebe Reiß- und Abriebfestigkeit, eine Kunststofffolie Wasser- und Winddichtigkeit, und die Verbindung mit einer Metallfolie ergibt ein lichtundurchlässiges Material, das unter Umständen auch noch Wärmestrahlung reflektiert. Die Außenschicht kann bspw. mit einer sehr dünnen Membranfolie laminiert sein, die winddicht, atmungsaktiv und/oder wasserdicht ist.

Bei den Beschichtungen können sowohl Faserbeschichtungen als auch Flächenbeschichtungen zum Einsatz kommen. Wird z.B. Teflon als Beschichtung eingesetzt (und nicht als Membran), werden die Fasern mit dem Kunststoff ummantelt.

Bei einer Flächenbeschichtung, welche meist auf ein Trägermaterial aufgedampft oder aufgestrichen wird, können einfache oder mikroporöse Flächenbeschichtungen erfindungsgemäß eingesetzt werden. Bei der einfachen Variante (wasserdicht aber nicht atmungsaktiv) wird die Gewebefläche geschlossen beschichtet, es kann kein Wasser mehr eindringen, aber es geht auch keine Feuchtigkeit heraus.

Bei den mikroporösen Membranen (wasserdicht und atmungsaktiv) ist der Beschichtungsfilm von winzigen Poren durchsetzt, die ein Eindringen von Feuchtigkeit verhindern, aber ein Austreten von Wasserdampf ermöglichen. Bei diesen Beschichtungen bleibt die Atmungsaktivität erhalten. Gleiche Charakteristika weisen die hydrophilen und hydrophoben Membranen auf.

Für die Verarbeitung von Membranen auf Textilien gibt es verschiedene Möglichkeiten: Beim Oberstofflaminat wird die Membran direkt mit dem Oberstoff als Zweilagen-Laminat verbunden. Beim Insertlaminat wird die Membran auf ein leichtes Trägermaterial laminiert und zwischen Oberstoff und Futter verarbeitet. Beim Futterlaminat wird der Futterstoff auf der linken Seite mit der Membran verbunden. Beim Dreischichtlaminat wird die Membran nicht nur mit dem Oberstoff, sondern auch mit dem Futterstoff verbunden.

Von der Erfindung umfasst sind sämtliche beschriebene sowie dem Fachmann bekannte Verarbeitungsverfahren zum Aufbringen einer Beschichtung und/oder einer Membran auf Textilien.

Eine erfindungsgemäß bevorzugte Beschichtung ist bspw. eine Kunststoff- und/oder Kunstharz Beschichtung, wie bspw. eine Polyurethan- (PU) oder Polyvinylchlorid (PVC) Beschichtung. Die PU-Beschichtungen haben den Vorteil, dass sie im Vergleich zu PVC-Beschichtungen leichter sind, auch bei niedrigen Außentemperaturen elastisch bleiben und umweltverträglicher sind.

In einer weiteren bevorzugten Ausführungsform handelt es sich bei der Membran vorzugsweise um eine mikroporöse Membran, insbesondere um eine synthetische und/oder halbsynthetische Polymermembran, vorzugsweise eine Polytetrafluoroethylen (PTFE) Membran, insbesondere eine gereckte Polytetrafluoroethylen (PTFE) Membran.

Die erfindungsgemäße wiederverwendbare Schwimmwindel zeichnet sich u.a. auch dadurch aus, dass die Beschichtung und/oder (mikroporöse) Membran im Wesentlichen ohne Nahtführung auf die Außenschicht aufgebracht ist. Ein Verfahren um solche nahtlosen Verbindungen herzustellen stellt bspw. das sogenannte Welding (Druckschweißen) dar. Alternative Verfahren sind bspw. das schon beschriebene Verkleben/Verschmelzen auf speziellen Textilmaschinen. Eine besonders bevorzugte wiederverwendbare Schwimmwindel zeichnet sich daher dadurch aus, dass die Beschichtung und/oder (mikroporöse) Membran mittels Welding (Druckschweißen) dauerhaft auf die Außenschicht aufgebracht ist. Generell ist darauf zu achten, dass nach Möglichkeit eine nahtfreie Verbindung zwischen Außenschicht, Beschichtung und/oder (mikroporöse) Membran vorliegt, da eine Naht ein perforieren erfordert und somit die Wasserdichtigkeit eingeschränkt wird.

Ergänzend hierzu kann jede Außenschicht noch einen Sonnenschutzfaktor, genauer einen UV-Schutzfaktor (USF) oder Ultraviolet Protection Factor (UPF) aufweisen. Bevorzugt weist die Außenschicht einen UPF > 15 auf, besonders bevorzugt von > UPF 40. Der Sonnenschutz wird auf die Außenschicht mittels Ein- oder Aufbringen von UV- absorbierenden oder -reflektierenden Agenzien, durch Verwendung besonders dicht gewebter Stoffe oder durch bewusst eingesetzter Farbauswahl, erwirkt.

In einer weiteren bevorzugten Ausführungsform ist die wasserabsorbierende Zwischenschicht mittels Welding (Druckschweißen) dauerhaft zwischen der Außen- und Innenschicht aufgebracht. Bevorzugt handelt es sich bei der wasserabsorbierenden Zwischenschicht um eine Frottee- und/oder Fleece-Zwischenschicht. Frottee bietet den Vorteil, dass es besondere Saugfähigkeit aufweist und gleichzeitig einen angenehmen Griff hat. Bevorzugt ist die wasserabsorbierende Zwischenschicht nur im Intimbereich angebracht.

In einer weiteren besonders bevorzugten Ausführungsform zeichnet sich die wiederverwendbare Schwimmwindel dadurch aus, das die wasserdampfdurchlässige Innenschicht aus einer Maschenware aus synthetischen Fasermaterialien besteht, vorzugsweise eine Polyester Innenschicht, insbesondere eine Polycarbonat (PC), Polyethylenterephthalat (PET) und/oder Polyesterharz Innenschicht.

Erfindungsgemäß werden unter Polyester alle Polymere mit Esterfunktionen -[-CO-O-]- in ihrer Hauptkette umfasst. Hierunter fallen bspw. die synthetische Polymere (Kunststoffe), zu denen die viel verwendeten Polycarbonate (PC), Polyethylenterephthalat (PET) und das duroplastische ungesättigte Polyesterharz (UP) gehören. Weitere Fasern aus synthetischen Polymeren die erfindungsgemäß umfasst sind, sind bspw. Polyamide (PA), Polyimide (PI), Polyamidimide (PAI), Polyphenylensulfide (PPS), Aramide, Polyacrylnitrile (PAN), Polytetrafluorethylene (PTFE), Polyethylene (PE), Polypropylene (PP), Polyvinylchloride (PVC bzw. bei Fasern CLF) und Polyurethane (EL).

Besonders bevorzugt ist die wasserdampfdurchlässige Innenschicht aus Materialien aufgebaut, die "one-directional wicking" Eigenschaften aufweisen. Erfindungsgemäß wird hierunter eine Weiterentwicklung des herkömmlichen "wicking" verstanden, bei dem der Feuchtigkeitsgehalt des Materials nicht nur bezüglich der Größe, sondern auch der Position geregelt wird. Das heißt, dass die auf der Stoffoberseite auftreffende Feuchtigkeit zuerst und schnell auf die Stoffunterseite gezogen wird, von wo dann die Wickingfunktion (=größtmögliches Verteilen der Feuchtigkeit) stattfindet. Daraus folgt, dass auf der Materialoberseite (bei der erfindungsgemäßen Schwimmwindel handelt es sich hierbei um die dem Körper zugewandte Materialseite) nur kleine Mengen an Restfeuchtigkeit verbleiben, während die überwiegende Menge an Feuchtigkeit auf die dem-Körper-abgewandte Seite abgezogen wird, um dort zu verdunsten. Im Vergleich dazu wird beim herkömmlichen Wicking der Feuchtigkeitsgehalt auf der Stoffober- und -unterseite gleichmäßig und über eine große Fläche verteilt, was zu einem entsprechend "größtmöglichen und flächigen" Kontakt der (sensiblen) (Klein)Kinderhaut mit der angestauten Feuchtigkeit führt und zu Hautirritationen, Rötungen, Reibungen, vermindertem Tragekomfort etc. führen kann. Materialien die entsprechende "one-directional-wicking" Eigenschaften aufweisen und demgemäß als wasserdampfdurchlässige Innenschicht verwendet werden können sind bspw. die beschriebenen synthetischen Fasermaterialien, insbesondere die Polyester Fasermaterialien. Dem Fachmann sind weitere Kunstfaser/- mischungen bekannt, die erfindungsgemäß zum Einsatz kommen können.

In einer weiteren bevorzugten Ausführungsform stellt das "o-ne-directional-wicking" ein sogenanntes "Finishing" dar, d.h. das auf die fertige Schicht, insbesondere wasserdampfdurchlässige Innenschicht, das fertige Futter mittels chemischmechanischer Verfahren aufgebracht wird. Dem Fachmann sind entsprechende Verfahren bekannt.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:
Fig. 1 Eine Darstellung einer Ausführungsform der wiederverwendbaren Schwimmwindel;
Fig. 2 Eine Gegenüberstellung von Materialien mit normaler "Wicking" und "one-directional-wicking" Eigenschaften;
Fig. 3 Darstellung einer geweldeten Frotteeinnenschicht;
Fig. 4 Schematische Darstellung des Schichtaufbaus der wiederverwendbaren Schwimmwindel.

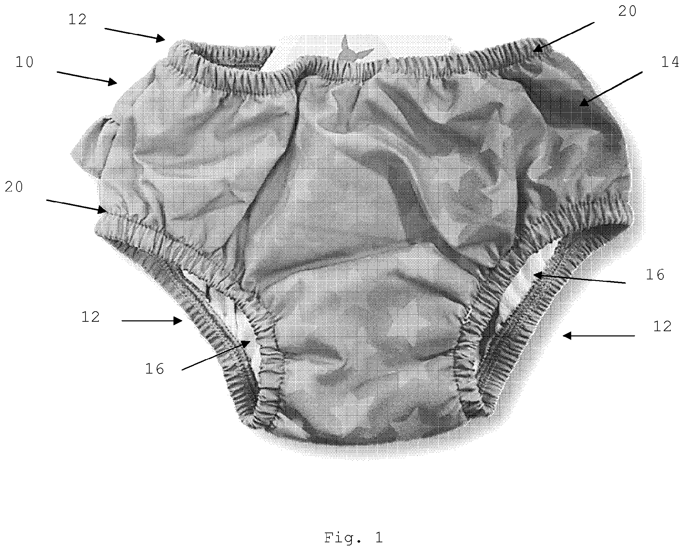

Fig. 1 zeigt eine Ausführungsform der wiederverwendbaren Schwimmwindel (10) in Form eines Höschens, mit einem elastischen Abschluss, vorliegend eines Gummizuges, im Taillen und/ Beinbereich (12) sowie eine Außenschicht (14) und eine Innenschicht (16). Ferner zeigt Fig. 1, dass der elastische Abschluss mittels einer Naht (18) an die wiederverwendbare Schwimmwindel (10) befestigt ist.

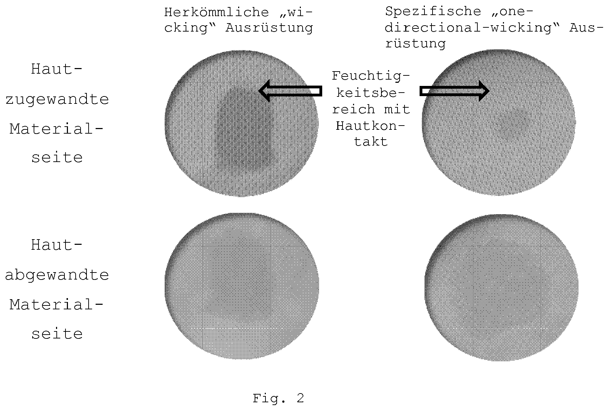

Fig. 2 zeigt Eine Gegenüberstellung von Materialien mit normaler "Wicking" und "one-directional-wicking" Eigenschaften. Deutlich ist zu erkennen, wie bei den "one-directional-wicking" Materialien die auf der Stoffoberseite auftreffende Feuchtigkeit zuerst und schnell auf die Stoffunterseite gezogen wird, von wo dann die Wickingfunktion (=größtmögliches Verteilen der Feuchtigkeit) stattfindet. Daraus folgt, dass auf der Materialoberseite (bei der erfindungsgemäßen Schwimmwindel handelt es sich hierbei um die dem Körper zugewandte Materialseite) nur kleine Mengen an Restfeuchtigkeit verbleiben, während die überwiegende Menge an Feuchtigkeit auf die dem-Körper-abgewandte Seite abgezogen wird, um dort zu verdunsten. Im Vergleich dazu wird beim herkömmlichen Wicking der Feuchtigkeitsgehalt auf der Stoffober- und -unterseite gleichmäßig und über eine große Fläche verteilt, was zu einem entsprechend "größtmöglichen und flächigen" Kontakt der Haut mit der angestauten Feuchtigkeit führt.

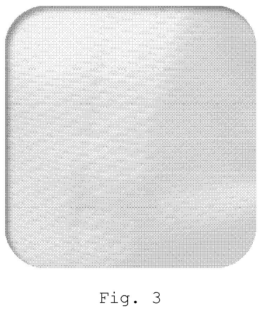

Fig. 3 zeigt einen zusätzlichen Feuchtigkeitsspeicher in Form eines geweldeten Frotteematerials. Dieses kann erfindungsgemäß als Zwischenschicht genutzt werden, wobei - wie weiter oben dargelegt - die Verbindung der Zwischenschicht mit der Außen- sowie der Innenschicht nahtlos, vorliegend mittels "Welding" (Druckschweißens) erfolgen kann.

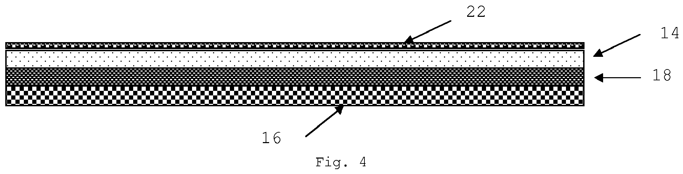

Fig. 4 zeigt eine schematische Darstellung des Schichtaufbaus der wiederverwendbaren Schwimmwindel. Gezeigt ist die Außenschicht (14), die Innenschicht (16), die Zwischenschicht (18) sowie eine Beschichtung (22), die auf die Außenschicht (14) aufgebracht ist und die wasserdichte gewährleisten soll. Ferner ist dargelegt, dass die Zwischenschicht (18) ohne Nahtführung zwischen der Außenschicht (14) und der Innenschicht (16) befestigt ist. Dies kann bspw. mittels Welding (Druckschweißen), Verkleben oder Verschmelzen auf speziellen Textilienmaschinen erfolgen. Die Beschichtung (22) ist ebenfalls ohne Nahtführung auf die Außenschicht (14) aufgebracht worden. Die Verbindung erfolgt dabei stoffschlüssig beispielsweise durch Verkleben oder Verschmelzen auf speziellen Textilmaschinen (Lamination) oder mittels Welding (Druckschweißen).

Es sind zahlreiche Abwandlungen und Weiterbildungen der beschriebenen Ausführungsbeispiele verwirklichbar.

### Bezugszeichen

- 10: wiederverwendbare Schwimmwindel
- 12: elastischer Abschluss
- 14: Außenschicht
- 16: Innenschicht
- 18: Zwischenschicht
- 20: Naht
- 22: Beschichtung

### zitierte Literatur

zitierte Patentliteratur
US7678094B1

## Patentansprüche

1. Wiederverwendbare Schwimmwindel, in Form eines Höschens, mit einem elastischen Abschluss im Taillen und/oder Beinbereich, umfassend:
a) eine Außenschicht, wobei die Außenschicht aus einer elastischen Maschenware oder einer Webware ausgestaltet ist;
b) einer wasserabsorbierenden Zwischenschicht, wobei die wasserabsorbierende Zwischenschicht aus einem Textilgewebe ausgestaltet ist;
c) eine wasserdampfdurchlässige Innenschicht, wobei die wasserdampfdurchlässige Innenschicht aus einer Maschenware aus synthetischen Fasermaterialien ausgestaltet ist;
d) und wobei die wasserabsorbierende Zwischenschicht im Wesentlichen ohne Nahtführung zwischen Außen- und Innenschicht aufgebracht ist.

2. Wiederverwendbare Schwimmwindel nach Anspruch 1, **dadurch gekennzeichnet, dass** die Außenschicht eine wasserdichte Beschichtung und/oder eine mikroporöse Membran aufweist.

3. Wiederverwendbare Schwimmwindel nach Anspruch 2, **dadurch gekennzeichnet, dass** es sich bei der Beschichtung um eine Kunststoff- und/oder Kunstharz Beschichtung handelt.

4. Wiederverwendbare Schwimmwindel nach Anspruch 3, **dadurch gekennzeichnet, dass** es sich bei der Beschichtung um eine Polyurethan-Beschichtung handelt.

5. Wiederverwendbare Schwimmwindel nach Anspruch 2, **dadurch gekennzeichnet, dass** es sich bei der mikroporösen Membran um eine synthetische und/oder halbsynthetische Polymermembran, vorzugsweise eine Polytetrafluoroethylen (PTFE) Membran, insbesondere eine gereckte Polytetrafluoroethylen (PTFE) Membran, handelt.

6. Wiederverwendbare Schwimmwindel nach einem der Ansprüche 2 bis 5, **dadurch gekennzeichnet, dass** die Beschichtung und/oder mikroporöse Membran im Wesentlichen ohne Nahtführung auf die Außenschicht aufgebracht ist.

7. Wiederverwendbare Schwimmwindel nach Anspruch 6, **dadurch gekennzeichnet, dass** die Beschichtung und/oder mikroporöse Membran mittels Welding (Druckschweißen), Verkleben und/oder Verschmelzen dauerhaft auf die Außenschicht aufgebracht ist.

8. Wiederverwendbare Schwimmwindel nach einem der vorhergehenden Ansprüche, **dadurch gekennzeichnet, dass** die wasserabsorbierende Zwischenschicht mittels Welding (Druckschweißen), Verkleben und/oder Verschmelzen dauerhaft zwischen Außen- und Innenschicht aufgebracht ist.

9. Wiederverwendbare Schwimmwindel nach einem der vorhergehenden Ansprüche, **dadurch gekennzeichnet, dass** es sich bei der wasserabsorbierenden Zwischenschicht um eine Frottee-Zwischenschicht handelt.

10. Wiederverwendbare Schwimmwindel nach einem der vorhergehenden Ansprüche, **dadurch gekennzeichnet, dass** es sich bei der wasserdampfdurchlässigen Innenschicht aus einer Maschenware aus synthetischen Fasermaterialien um eine Polyester Innenschicht, vorzugsweise eine Polycabonat (PC), Polyethylenterephthalat (PET) und/oder Polyesterharz, handelt.

## Claims

1. Reusable swimming nappy, in the form of a brief, having an elastic termination in the waist and/or leg region, **comprising**
a) an outer layer, wherein the outer layer is constructed from an elastic knitted fabric or a woven fabric;
b) a water-absorbing interlayer, wherein the water-absorbing interlayer is constructed from a woven textile fabric;
c) a water vapour pervious inner layer, wherein the water vapour pervious inner layer is constructed from a knitted fabric formed of synthetic fibrous materials;
d) and wherein the water-absorbing interlayer is applied between the outer and the inner layers essentially without seam guide.

2. Reusable swimming nappy according to Claim 1, **characterized in that** the outer layer includes a waterproof coating and/or a microporous membrane.

3. Reusable swimming nappy according to Claim 2, **characterized in that** the coating is a coating with manufactured polymer and/or synthetic resin.

4. Reusable swimming nappy according to Claim 3, **characterized in that** the coating is a polyurethane coating.

5. Reusable swimming nappy according to Claim 2, **characterized in that** the microporous membrane is a synthetic and/or semisynthetic polymeric membrane, preferably a polytetrafluoroethylene (PTFE) membrane, especially an expanded polytetrafluoroethylene (PTFE) membrane.

6. Reusable swimming nappy according to any one of Claims 2 to 5, **characterized in that** the coating and/or microporous membrane is applied to the outer layer essentially without seam guide.

7. Reusable swimming nappy according to Claim 6, **characterized in that** the coating and/or microporous membrane is applied to the outer layer in a durable manner by welding, adhering and/or fusing.

8. Reusable swimming nappy according to any preceding claim, **characterized in that** the water-absorbing interlayer is applied between the outer and inner layers in a durable manner by welding, adhering and/or fusing.

9. Reusable swimming nappy according to any preceding claim, **characterized in that** the water-absorbing interlayer is a terry towelling interlayer.

10. Reusable swimming nappy according to any preceding claim, **characterized in that** the water vapour pervious inner layer formed from a knitted fabric formed of synthetic fibrous materials is a polyester inner layer, preferably a polycarbonate (PC), polyethylene terephthalate (PET) and/or polyester resin inner layer.

## Revendications

1. Couche réutilisable pour la piscine, sous la forme d'une culotte, avec une fermeture élastique à la taille et/ou aux jambes, comprenant :
a) une couche extérieure, la couche extérieure étant configurée à partir d'un tricot élastique ou d'un tissu ;
b) une couche intermédiaire absorbant l'eau, la couche intermédiaire absorbant l'eau étant configurée à partir d'un tissu textile ;
c) une couche intérieure perméable à la vapeur d'eau, la couche intérieure perméable à la vapeur d'eau étant configurée à partir d'un tricot en matériaux fibreux synthétiques ;
d) la couche intermédiaire absorbant l'eau étant appliquée essentiellement sans couture entre la couche extérieure et intérieure.

2. Couche réutilisable pour la piscine selon la revendication 1, **caractérisée en ce que** la couche extérieure comprend un revêtement étanche à l'eau et/ou une membrane microporeuse.

3. Couche réutilisable pour la piscine selon la revendication 2, **caractérisée en ce que** le revêtement est un revêtement en plastique et/ou résine synthétique.

4. Couche réutilisable pour la piscine selon la revendication 3, **caractérisée en ce que** le revêtement est un revêtement en polyuréthane.

5. Couche réutilisable pour la piscine selon la revendication 2, **caractérisée en ce que** la membrane microporeuse est une membrane polymère synthétique et/ou semi-synthétique, de préférence une membrane en polytétrafluoroéthylène (PTFE), notamment une membrane en polytétrafluoroéthylène (PTFE) orienté.

6. Couche réutilisable pour la piscine selon l'une quelconque des revendications 2 à 5, **caractérisée en ce que** le revêtement et/ou la membrane microporeuse sont appliqués essentiellement sans couture sur la couche extérieure.

7. Couche réutilisable pour la piscine selon la revendication 6, **caractérisée en ce que** le revêtement et/ou la membrane microporeuse sont appliqués de manière durable sur la couche extérieure par soudage (soudage avec pression), collage et/ou fusion.

8. Couche réutilisable pour la piscine selon l'une quelconque des revendications précédentes, **caractérisée en ce que** la couche intermédiaire absorbant l'eau est appliquée de manière durable entre la couche extérieure et intérieure par soudage (soudage avec pression), collage et/ou fusion.

9. Couche réutilisable pour la piscine selon l'une quelconque des revendications précédentes, **caractérisée en ce que** la couche intermédiaire absorbant l'eau est une couche intermédiaire en éponge.

10. Couche réutilisable pour la piscine selon l'une quelconque des revendications précédentes, **caractérisée en ce que** la couche intérieure perméable à la vapeur d'eau en un tricot de matériaux fibreux synthétiques est une couche intérieure en polyester, de préférence en polycarbonate (PC), polyéthylène téréphtalate (PET) et/ou résine de polyester.
